(19) Europäisches Patentamt
European Patent Office
Office européen des brevets

(11) **EP 4 563 168 A1**

(12) **EUROPEAN PATENT APPLICATION**
published in accordance with Art. 153(4) EPC

(43) Date of publication:
04.06.2025 Bulletin 2025/23

(51) International Patent Classification (IPC):
**A61L 31/10** (2006.01)

(21) Application number: 23845736.0

(22) Date of filing: 31.07.2023

(52) Cooperative Patent Classification (CPC):
**A61L 31/10**

(86) International application number:
**PCT/CN2023/110241**

(87) International publication number:
**WO 2024/022532 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 29.07.2022 CN 202210907948

(71) Applicant: **Biotyx Medical (Shenzhen) Co., Ltd.
Shenzhen, Guangdong 518000 (CN)**

(72) Inventors:
• **LI, Yunfei
Shenzhen, Guangdong 518000 (CN)**

• **ZHANG, Deyuan
Shenzhen, Guangdong 518000 (CN)**
• **QI, Haiping
Shenzhen, Guangdong 518000 (CN)**
• **ZHANG, Wanqian
Shenzhen, Guangdong 518000 (CN)**
• **LI, Haifeng
Shenzhen, Guangdong 518000 (CN)**

(74) Representative: **Prinz & Partner mbB
Patent- und Rechtsanwälte
Rundfunkplatz 2
80335 München (DE)**

(54) **COATING FOR MEDICAL DEVICE**

(57)     A coating for a medical device. The coating includes a drug-loaded layer and a drug-controlled release layer covering the drug-loaded layer. A molecular weight retention rate m of a polymer in the drug-controlled release layer, a weight-average molecular weight $Mw_{controlled\ release}$ of the polymer, and the thickness $X_{controlled\ release}$ of the polymer meet the following relational expression;

$$k \times \frac{\sqrt{\left|\frac{A_1+A_2}{Mw_{controlled\ release}+A_2}-1\right|}}{m} - b \leq X_{controlled\ release} \leq k \times$$

$$\frac{\sqrt{\left|\frac{A_1+A_2}{Mw_{controlled\ release}+A_2}-1\right|}}{m} + b;$$

where $A_1$, $A_2$, k, and b are all fitting constants. The design of the coating may not only improve the release performance of a drug, but also effectively control the corrosion of a matrix so that the matrix of a degradable medical device cannot be degraded or can be slowly degraded within 1-6 months, and can be quickly degraded after 6 months.

EP 4 563 168 A1

Fig.2-1

Fig.2-2  Fig.2-3

FIG. 2

## Description

### Technical Field

The present invention belongs to the technical field of medical devices, particularly relates to a coating for a medical device, and more particularly relates to a coating for a degradable medical device.

### Background Art

Medical devices, especially implanted or interventional medical devices, are required not only to have basic therapeutic functions, but also to load drugs on their surfaces to prevent some reactions such as rejection, proliferation, thrombosis, and inflammation at the site where the device is implanted. Therefore, common medical devices nowadays usually have coatings added to their surfaces to load drugs or control drug release. For example, a vascular stent, after being implanted into the vessel, must not only expand the vessel and provide sufficient support for a certain period to ensure that the vessel remains unblocked, but also prevent vascular proliferation, thrombosis at the implant site, and subsequent restenosis. Therefore, vascular stents available on the market usually have one or more coatings added to their surfaces to load drugs and control drug release, thereby preventing abnormal reactions after the stent is implanted in the human body, which may affect its basic therapeutic effect.

The coating on the surface of the device is strictly required in terms of loading drugs and drug release. It is necessary to control the drug loading and drug release rate. If the drug loading is too large, too much drug in the human body will bring great toxic side effects and increase the risk of drug toxicity in the body. If the drug loading is too small, an effective prevention and treatment effect cannot be achieved. When the total drug loading of the medical device is constant, if the drug release rate is too slow, the drug is insufficient in the whole drug release stage, and the prevention and treatment effect of the drug is limited. If the drug release rate is too quick, the local drug concentration may become excessively high, leading to toxic side effects. Moreover, if the drug is released too quickly at the initial stage, the drug cannot be released continuously and effectively, which will lead to an insufficient amount of drug released at the later stage. In both cases, proliferation will occur at the implant site, eventually causing vascular restenosis. It can be seen that drug loading and drug-controlled release on the surface of the medical device are key difficulties in designing drug coatings. For a degradable medical device, the coating not only needs to load drugs and control drug release, but also plays a role in controlling the corrosion of the device matrix. Therefore, the design of the coating for the degradable device is more difficult and challenging.

Currently, although a large number of research results related to drug coatings have been disclosed, most of the existing coatings for medical devices only consider drug release, but do not consider the effect of the coating on the corrosion of the device substrate. Thus, none of the currently disclosed drug coating designs are generally suitable for use in degradable medical devices. Currently, some commonly used coatings are not yet perfect in terms of drug release and have some defects. In addition, when considering the corrosion requirement of the biodegradable medical device in the design of the coating, the type and molecular weight of polymers in the coating will change greatly. Different types and molecular weights of polymers will have very large differences in drug-controlled release, and thus the design scheme of the whole coating will change greatly. For example, in patent CN107496996B, a drug coating of a vascular stent is disclosed. The coating includes 3 layers, and each layer contains a drug and a drug carrier. An outer coating plays a role in controlling the release of an inner coating so that the burst release of the drug in the inner coating becomes smaller, thereby reducing the possibility of an acute toxicity reaction. However, the outer drug coating is also loaded with 2 drugs, and the molecular weight of the poly(lactic-co-glycolic acid) (PLGA) loaded with the drug is relatively small, only around 2,000. Meanwhile, the mass fraction ratio of rapamycin, curcumin, and the PLGA carrier loaded in the outer coating is 5: 2: 13. The outer coating has a low molecular weight of polymer, a low proportion of polymer, and a high proportion of drug. The whole coating design does not have a good sustained-release effect on the drug in the outer layer, and a large amount of rapamycin loaded in the outer layer will exhibit burst release. The middle layer and the outer layer are loaded with a large amount of rapamycin, which may result in the drug loading significantly exceeding the requirements, causing additional toxic side effects to the human body. In addition, the substrate of the vascular stent is stainless steel, so the design of the drug coating in this patent application does not need to consider the degradation of the matrix. Therefore, the polymer used is a polymer with a small molecular weight, which is not suitable for the medical device with a degradable substrate.

### Summary of the Invention

Based on this, the present application provides a coating for a medical device, especially a coating for a degradable medical device. The coating, through a unique design, ensures that the drug loading is within a suitable effective range, provides excellent performance in terms of drug-controlled release, and effectively controls the corrosion of a matrix.

The technical solution of the present invention provides a coating for a medical device, which has very excellent performance in controlling drug release. The special design of the coating is more inclusive of the processes of preparing the coating, solvents used in the preparation of the coating, and the crystal form and size of the drug in the final coating.

In the above-mentioned technical solution of the present invention, the coating includes a drug-loaded layer and a drug-controlled release layer covering the drug-loaded layer, and each of the drug-loaded layer and the drug-controlled release layer includes a polymer; a molecular weight retention rate m of the polymer in the drug-controlled release layer, a weight-average molecular weight $Mw_{controlled\ release}$ of the polymer, and a thickness $X_{controlled\ release}$ of the polymer meet the following relational expression:

$$\mathrm{k} \times \frac{\sqrt{\left|\frac{A_1+A_2}{Mw_{controlled\ release}+A_2}-1\right|}}{m} - b \leq X_{controlled\ release} \leq \mathrm{k} \times$$

$$\frac{\sqrt{\left|\frac{A_1+A_2}{Mw_{controlled\ release}+A_2}-1\right|}}{m} + b;$$

wherein $A_1$ is a fitting constant of 823;

$A_2$ is a fitting constant of 79.9;

k is a fitting constant of 1.5;

b is a fitting constant with a range of $0 \leq b \leq 2$;

$Mw_{controlled\ release}$ is the weight-average molecular weight of the polymer in the drug-controlled release layer in kDa, and X is in $\mu$m.

It should be particularly noted that b in the formula of the above-mentioned technical solution may be any value within the range of 0-2. For example, in some examples of the present invention, b may be any value within the range of 0.2, 0.5, 0.55, 0.6, 0.65, 0.7, 0.8, 0.9, 1.0, 1.1, 1.2, 1.3, 1.4, 1.5, 1.6, 1.7, 1.8, 1.85, 1.9, 1.95, or 2.0. In other examples of the present invention, b may be an interval consisting of any two values within the range of 0-2, such as 0.2-1.9, 0.5-1.5, 0.3-1.6, 0.3-1.2, 0.55-1.95, 0.6-1.95, 0.6-1.9, 0.6-1.85, 0.6-1.8, 0.6-1.7, 0.7-1.8, 0.75-1.6, 0.8-1.95, 0.8-1.9, 0.8-1.85, 0.8-1.8, 0.8-1.7, or 0.8-1.6.

According to a large number of experimental records, it is found in the present invention that there is an internal correlation or regularity among three variables of coating thickness, polymer type, and polymer molecular weight of the outer drug-controlled release layer. It is determined that there will be a relatively optimal polymer thickness range corresponding to a particular polymer and its particular molecular weight in the outer drug-controlled release layer, and the performance of the coating in terms of drug release may be greatly improved within this optimal polymer thickness range. In the present invention, the relational expression among the optimal thickness range of the drug-controlled release layer, the polymer type, and the polymer molecular weight is summarized through a large number of experimental results and innovations. That is, in the present application, through the design of the above-mentioned special outer drug-controlled release layer, the requirements for the process of the drug in the drug coating, the solvent used, and the final drug crystal form are correspondingly relaxed, and the rate and curve of drug release basically meet the requirements of corresponding medical devices.

The above-mentioned coating for a medical device provided by the present invention is a relatively universal drug coating suitable, which is suitable for drug-controlled release in a degradable medical device and a non-degradable medical device. Preferably, the above-mentioned coating is suitable for a degradable medical device.

According to the above-mentioned technical solution provided by the present invention, the weight-average molecular weight $Mw_{controlled\ release}$ of the polymer in the drug-controlled release layer is 20-900 kDa. In the technical solution of the present invention, the molecular weight of the polymer in the drug-controlled release layer may be any one of 20-900 kDa. In some examples, the molecular weight of the polymer in the drug-controlled release layer is 21 kDa. In other examples, the molecular weight of the polymer in the drug-controlled release layer is 898 kDa. In other examples, the molecular weight of the polymer in the drug-controlled release layer is 800 kDa. In still other examples, the molecular weight of the polymer in the drug-controlled release layer is 700 kDa.

According to the above-mentioned technical solution provided by the present invention, the molecular weight retention rate m of the polymer in the drug-controlled release layer is 50%-80%. In the present invention, the molecular weight retention rate m of the polymer refers to a retention rate of the molecular weight of the polymer when the polymer coating is placed in a PBS solution (pH = 7.4) with a concentration of 37°C for 1 month, i.e., the molecular weight retention rate m = $Mw_{controlled\ release\ in\ one\ month}$ /$Mw_{controlled\ release}$ × 100%, where $Mw_{controlled\ release\ in\ one\ month}$ refers to the molecular weight of the polymer when the polymer coating is placed in the PBS solution (pH = 7.4) with a concentration of 37°C for 1 month, and $Mw_{controlled\ release}$ is the molecular weight of the polymer in the drug-controlled release layer. The molecular weight retention rates of polymers at the same time point under the same condition are different. That is, in the present application, each molecular weight retention rate represents one specific polymer, and different molecular weight retention rates represent different polymers.

In the present invention, there may be one or multiple drug-loaded layers, and there may be one or multiple drugs loaded in the same drug-loaded layer. For example, in some examples, there is one drug-loaded layer with one loaded drug. In other examples, there are three drug-loaded layers with each layer loaded with a different drug. In other examples, there are two drug-loaded layers, where one layer is loaded with one drug and the other layer is loaded with two different drugs, i.e., the drug-loaded layers are loaded with a total of three drugs. In still other examples, there are two drug-loaded layers, where one layer is loaded with two drugs and the other layer is loaded with two different drugs, where the two drugs loaded in each of the two layers may be completely different, partially same, or identical.

According to the above-mentioned technical solution provided by the present invention, a drug-to-polymer mass ratio in the drug-loaded layer is 1: 0.1-50. Further, the drug-to-polymer mass ratio in the drug-loaded layer is 1: (0.1-30) or 1: 0.1-20. Furthermore, the drug-to-polymer mass ratio in the drug-loaded layer is 1: (0.2-18), 1: (0.5-18), 1: (0.6-15), 1: (0.8-18), 1: (0.8-16), 1: (0.8-15), 1: (0.5-12), 1: (0.5-8), 1: (0.5-6), 1: (0.2-16), 1: (0.2-12), 1: (0.1-12), 1: (0.2-10), 1: (0.1-10), 1: (0.2-8), 1: (0.1-8), 1: (0.2-5), 1: (0.2-4), 1: (0.2-3), 1: (0.1-5), 1: (0.1-4), or 1: (0.5-4).

According to the above-mentioned technical solution provided by the present invention, drug particles in the drug-loaded layer have a particle size r ≤ 0.8 μm. The crystal form of the drug in the drug-loaded layer may be crystalline, non-crystalline, or semi-crystalline. That is, in some examples of the present invention, the drug in the drug-loaded layer is crystalline, in other examples, the drug in the drug-loaded layer is non-crystalline, and in still other examples, the drug in the drug-loaded layer is semi-crystalline. Due to the design of the special outer coating provided in the present invention, there is no too high requirement for the crystal form or crystal size of the drug in the drug-loaded layer in the present invention. That is, regardless of whether the drug in the drug-loaded layer is crystalline, non-crystalline, or semi-crystalline, when the particle size of the drug is controlled below 0.8 μm, the drug release rate can meet the corresponding requirement. Therefore, in the present invention, through the special design of the outer drug-controlled release layer and the drug-loaded layer, the requirements for the process of loading a drug in the drug-loaded layer and the solvent are relatively greatly reduced so that for the drug coatings formed through different processes and solvents, as long as the particle size of the drug is not very large and the dispersion is relatively uniform, the drug release rate is within a relatively desirable range.

In some examples of the present invention, the drug particles in the drug-loaded layer have a particle size r ≤ 0.8 μm. In other examples of the present invention, the drug particles in the drug-loaded layer have a particle size r ≤ 0.6 μm. In still other examples of the present invention, the drug particles in the drug-loaded layer have a particle size r ≤ 0.45 μm.

It should be noted that in the present invention, the particle size refers to an average particle size. For example, "the drug particles in the drug-loaded layer have a particle size r of 0.6 μm" refers to that the drug particles in the drug-loaded layer have an average particle size of 0.6 μm, where there are some drug particles having a particle size greater than 0.6 μm and some drug particles having a particle size less than 0.6 μm.

In addition, it should be noted that when the drug in the drug-loaded layer is crystalline, the particle size of the drug particle is a particle size of the crystal form, and when the drug in the drug-loaded layer is non-crystalline or semi-crystalline, the particle size of the drug particle is a particle size of a drug cluster dispersed in the polymer.

According to the above-mentioned technical solution provided by the present invention, a sum of thicknesses of the drug-loaded layer and the drug-controlled release layer is not less than 2.5 μm. Further, the sum of the thicknesses of the drug-loaded layer and the drug-controlled release layer is not less than 5 μm. Furthermore, the sum of the thicknesses of the drug-loaded layer and the drug-controlled release layer is within (5μm, 38μm]. In some examples of the present invention, the sum of the thicknesses of the drug-loaded layer and the drug-controlled release layer is 5.1 μm, 5.5 μm, 5.2 μm, or 6 μm. In some examples of the present invention, the sum of the thicknesses of the drug-loaded layer and the drug-controlled release layer is 7 μm, 7.5 μm, or 8 μm.

According to the above-mentioned technical solution provided by the present invention, the polymer in the drug-loaded layer has a molecular weight of 30-1,000 kDa. Further, polymers in the inner and middle layers have a molecular weight of 40-1,000 kDa, 50-1,000 kDa, 60-1,000 kDa. Furthermore, the polymer in the drug-loaded layer has a molecular weight of 70-1,000 kDa , 80-1,000 kDa.

According to the above-mentioned technical solution provided by the present invention, an inner side of the drug-loaded layer further includes a corrosion control layer, and a polymer in the corrosion control layer has a molecular weight of 50-1,000 kDa. Further, polymers in the inner and middle layers have a molecular weight of 60-1,000 kDa, 70-1,000 kDa, 80-1,000 kDa. Furthermore, the polymer in the drug-loaded layer has a molecular weight of 90-1,000 kDa , 100-1,000 kDa.

In the above-mentioned technical solution provided by the present invention, the molecular weight of the polymer in the drug-controlled release layer may fluctuate in a wide range, and the drug-loaded layer and the corrosion control layer have higher requirements for the molecular weight of the polymer than the drug-controlled release layer. The polymer in the drug-loaded layer is a carrier of an active drug, and its type, molecular weight, drug-loaded coating thickness, and mass ratio to the drug have important effects on the drug release rate. The higher the molecular weight of the polymer, the more obvious the phase separation between the polymer and the drug, and the quicker the drug release rate. The smaller the ratio of the polymer mass to the drug mass, the weaker the binding ability of the polymer to the drug, and the quicker the drug release rate. The thinner the drug-loaded coating, the shorter the drug diffusion release path, and the quicker the drug release rate. In the corrosion control layer, the molecular weight of the polymer is relatively high, the molecular weight is too small, and the corrosion rate is quick in the early stage, which easily leads to the rapid corrosion of the degradable metal part of the device in the early stage and does not meet the requirements of mechanical performance, or leads to that the rapid corrosion of the degradable metal part of the device cannot be effectively regulated in the later stage after the premature degradation of the polymer.

According to the above-mentioned technical solution provided by the present invention, a total thickness of the corrosion control layer, the drug-loaded layer, and the drug-controlled release layer is 3.5-45 $\mu$m. Further, the total thickness of the corrosion control layer, the drug-loaded layer, and the drug-controlled release layer is 5.5-40 $\mu$m. Furthermore, the total thickness of the corrosion control layer, the drug-loaded layer, and the drug-controlled release layer is 5.8-36 $\mu$m.

According to the above-mentioned technical solution provided by the present invention, a thickness ratio of the corrosion control layer, the drug-loaded layer, and the drug-controlled release layer is 1: (0.5-15): (0.2-13).

According to the above-mentioned technical solution provided by the present invention, the thickness of the drug-controlled release layer is 0.2-8 $\mu$m. Further, the thickness of the corrosion control layer is 0.5-7 $\mu$m. Furthermore, the thickness of the corrosion control layer is 0.8-6.6 $\mu$m.

According to the above-mentioned technical solution provided by the present invention, the thickness of the corrosion control layer is 0.2-6 $\mu$m. Further, the thickness of the corrosion control layer is 0.5-5 $\mu$m. Furthermore, the thickness of the corrosion control layer is 0.8-3 $\mu$m.

According to the above-mentioned technical solution provided by the present invention, the thickness of the drug-loaded layer is 1-8 $\mu$m. Further, the thickness of the corrosion control layer is 1.5-7 $\mu$m. Furthermore, the thickness of the corrosion control layer is 1.5-6 $\mu$m.

According to the above-mentioned technical solution provided by the present invention, at least one layer of the three-layer structure of the coating further includes a corrosion promoter, a corrosion retardant, or an antioxidant.

According to the above-mentioned technical solution provided by the present invention, the coating further includes a metal isolation layer located on an inner side the corrosion control layer. The metal isolation layer is a coating containing a metal element more active than a medical device substrate, and the metal coating may be densely and uniformly distributed on the surface of the degradable device substrate by electroplating, spraying, or chemical plating.

According to the above-mentioned technical solution provided by the present invention, the metal isolation layer may be configured to delay the corrosion of a matrix of the medical device.

In some examples of the present invention, the electronegativity of at least one metal in the metal isolation layer is less than the electronegativity of the matrix metal of the degradable medical device, i.e., at least one metal in the metal isolation layer is more active than the matrix metal of the degradable medical device. The metal isolation layer may be a pure metal or a metal alloy. When the metal isolation layer is a pure metal, the electronegativity of the metal is less than the electronegativity of the matrix metal of the degradable medical device, that is, the metal is more active than the matrix metal of the degradable medical device. For example, when the matrix of the degradable medical device is iron-based, the metal isolation layer is pure zinc or pure magnesium. When the metal isolation layer is a metal alloy, main metal elements in the metal alloy, i.e., one or more elements with a high content, are more active or less electronegative than the matrix metal of the degradable medical device. For example, when the matrix of the degradable medical device is iron-based, the metal isolation layer is a zinc alloy or a magnesium alloy. In other examples of the present invention, the metal isolation layer may be a metal oxide-containing isolation layer. In other examples of the present invention, the metal isolation layer may be any other metal-containing coating that prevents corrosion of the matrix of the medical device.

According to the above-mentioned technical solution provided by the present invention, when the metal in an absorbable metal matrix is pure iron or an iron-based alloy, the metal isolation layer includes pure magnesium, a magnesium-containing alloy, pure zinc, or a zinc-containing alloy. When the metal in the absorbable metal matrix is pure zinc or a zinc-containing alloy, the metal isolation layer includes pure magnesium and a magnesium-containing alloy.

Since the metal in the metal isolation layer is more active than the metal constituting the device substrate, electrochemical protection may be formed in the in vivo microenvironment. The relatively active metal in the isolation layer preferentially corrodes, while the device substrate playing a supporting role is protected to delay corrosion. For example, when the device substrate is iron or an iron alloy, the surface of the device is covered with a metal coating including zinc or a zinc alloy, and a galvanic cell may be formed in the in vivo microenvironment. The metal coating including zinc or the zinc alloy acts as a negative electrode, loses electrons to become zinc ions, and is preferentially corroded. The iron or iron alloy device substrate is temporarily protected from corrosion as a positive electrode.

In the present invention, the four schemes of the drug-controlled release layer and the drug-loaded layer, or the drug-controlled release layer, the drug-loaded layer, and the corrosion control layer, or the drug-controlled release layer, the drug-loaded layer, and the metal isolation layer, or the drug-controlled release layer, the drug-loaded layer, the corrosion control layer, and the metal isolation layer may all control the drug release and the corrosion of the substrate. The various parameters of the layers of the coating in each scheme may cooperate and match with each other to realize the object of the present invention. For example, in some examples of the present invention, the coating for a medical device is the drug-controlled release layer and the drug-loaded layer, or the drug-controlled release layer, the drug-loaded layer, and the corrosion control layer from the outside to the inside. Therefore, the final coating has excellent drug release performance and excellent corrosion-regulating performance by adjusting the polymer type, the polymer molecular weight, the coating thickness, and the drug loading in each layer of the above-mentioned coating within corresponding intervals so that after implanting the device into a corresponding site, there will be neither proliferation nor inflammation, while there will be no corrosion or slow corrosion in the first 1-6 months and rapid corrosion after 6 months.

According to the above-mentioned technical solution provided by the present invention, a thickness of the metal isolation layer is $\geq 0.6\ \mu$m. Further, the thickness of the metal isolation layer is between 0.6-3.8 $\mu$m. When the thickness of the metal isolation layer is sufficient, the premature corrosion of the matrix metal of the degradable medical device may be effectively prevented to ensure that the matrix is not corroded in the first 1-6 months. However, the thickness of the metal isolation layer shall not be too large. Otherwise, the metal isolation layer cannot be corroded completely within 2 years, which finally results in that the degradable medical device cannot be quickly corroded after 6 months and cannot be completely corroded within 2 years.

According to the above-mentioned technical solution provided by the present invention, the polymer in the coating is at least one of a degradable polyester or a degradable polyanhydride. Further, in the present invention, the polymer is selected from at least one of poly(racemic lactic acid), poly(L-lactic acid), poly(D-lactic acid), poly(hydroxyethyl ester), polyurethane, polyamino acid, poly(lactic-co-diglycolic acid), poly-D,L-lactide, polypropylene glycol, polyglycolic acid, poly(lactic-co-glycolic acid, polysalicylic anhydride ester, polytrimethylene carbonate, polycaprolactone, poly($\varepsilon$-caprolactone), polyhydroxyalkanoate, polyacrylate, polysuccinate, poly($\beta$-hydroxybutyrate), polyethylene glycol adipate, poly(1,3-bis(p-carboxyphenoxy)propane-sebacic acid), polyerucic acid dimer-sebacic acid, and polyfumaric acid-sebacic acid. In some examples of the present invention, the polymer in the coating may be a specific one of the above-mentioned polymers. For example, the polymers in the drug-controlled release layer, the corrosion control layer, and the corrosion control layer in the coating are all poly(racemic lactic acid). In other examples of the present invention, the polymer in the coating may be a mixture of two or more of the above-mentioned polymers, such as a mixture of two or more of poly(L-lactic acid), poly(D-lactic acid), polyurethane, polysalicylic anhydride ester, polytrimethylene carbonate, or polycaprolactone. The mixture of two or more polymers may refer to one layer containing two polymers at the same time or each single layer containing only a polymer which is different from the polymer in another layer. For example, in some examples of the present invention, the polymer in the outer drug-controlled release layer in the coating is poly(L-lactic acid), the polymer in the drug-loaded layer is poly(racemic lactic acid), and the polymer in the corrosion control layer is polyurethane. In other examples of the present invention, the polymers in drug-controlled release layer, the corrosion control layer, and the corrosion control layer in the coating are all polyurethane and polysalicylic anhydride ester, or a mixture of poly(racemic lactic acid), poly(L-lactic acid), and poly(D-lactic acid). In still other examples, the polymers in the drug-controlled release layer and the corrosion control layer of the coating are poly(racemic lactic acid), and the polymer in the corrosion control layer is a mixture of polyhydroxyalkanoate and polyacrylate.

According to the above-mentioned technical solution provided by the present invention, a drug loaded in the coating is at least one of a drug inhibiting vascular proliferation, an anti-inflammatory drug, an antithrombotic drug, and a sensitizing drug. The antithrombotic drug includes an antiplatelet drug and an anticoagulant drug. In some examples of the present invention, the drug loaded in the coating is only the drug inhibiting vascular proliferation. In other examples, the drug loaded in the coating is a combination of any two or more of the drug inhibiting vascular proliferation, the anti-inflammatory drug, the antithrombotic drug, and the sensitizing drug. More specifically, in the present invention, the drug inhibiting vascular proliferation is selected from at least one of paclitaxel, rapamycin, and derivatives thereof; the antiplatelet drug includes cilostazol; the antithrombotic drug includes heparin; the anti-inflammatory drug is dexamethasone; the anti-sensitizing drug is selected from at least one of calcium gluconate, chlorpheniramine, and cortisone.

According to the above-mentioned technical solution provided by the present invention, the drug loaded in the drug-loaded layer is at least one of paclitaxel, rapamycin, cilostazol, heparin, tacrolimus, everolimus, dexamethasone, calcium gluconate, chlorpheniramine, or cortisone.

According to the above-mentioned technical solution provided by the present invention, the medical device is a degradable metal medical device or a degradable non-metal medical device, and the metal is a pure metal or a metal alloy. Further, the degradable medical device is a medical device including, at least in part, an iron base, a zinc base, a polymer, and a magnesium base. In some examples of the present invention, the matrix of the degradable medical device includes one metal or metal alloy. For example, the whole degradable medical device is an iron-based medical device. In other examples of the present invention, the matrix of the degradable medical device includes two or more materials, such as being partly iron-based and partly zinc-based, or partly metal-based and partly non-metal-based.

According to the above-mentioned technical solution provided by the present invention, the medical device includes any one of a vascular stent, a heart valve, a non-vascular endoluminal stent, an occluder, an orthopedic implant, a dental implant, a respiratory implant, a gynecological implant, an andrological implant, a suture, or a bolt.

According to the above-mentioned technical solution provided by the present invention, the stent of the present invention is the vascular stent or the heart valve stent. Further, the stent of the present invention is a balloon-expandable stent or a self-expanding stent. Furthermore, the stent of the present invention includes a coronary stent, a below-knee stent, a superficial femoral stent, an intracranial stent, a carotid stent, a pulmonary artery stent, or a renal artery stent.

According to the above-mentioned technical solution provided by the present invention, the substrate of the degradable medical device is pure iron or an iron-based alloy with a carbon content of not more than 2.11 wt%.

It should be particularly noted that metals in the above-mentioned iron, zinc, or magnesium bases all refer to the main components in the substrate. For example, the iron base includes pure iron or iron alloy, i.e., the iron base means that the main component in this kind of substrate is iron.

In the present invention, the release of the drug in the drug-loaded layer is controlled by the relationship or inherent logic among the type of polymer, the molecular weight of the polymer, and the thickness of the polymer of the drug-controlled release layer. The drug-controlled release layer provided in the present invention may effectively prevent the situation that the drug in the drug-loaded layer from being rapidly released under the flushing of blood immediately after the stent is implanted in the human body, which results in too quick drug release in the early stage and insufficient drug release in the later stage, so as to ensure a sustained and effective drug release, thereby avoiding the vascular restenosis. That is, the drug-controlled release layer in the present invention has a certain protective effect on the drug-loaded layer and may regulate the release rate of the drug loaded in the drug-loaded layer. In the present invention, a special drug-controlled release layer is added outside the drug-loaded layer so that the drug in the drug-loaded layer will gradually increase the contact area of the drug-loaded layer with blood only after the polymer in the drug-controlled release layer has been degraded to a certain extent, thereby effectively controlling the problem of burst release of the drug at the early stage of stent implantation.

The drug-controlled release layer and the drug-loaded layer in the present invention cooperate to control the drug release. Therefore, the release rate of the drug at each stage is effectively controlled, thereby ensuring that the amount of the drug released at each stage just meets the demand. In addition, the drug-loaded layer is loaded with an appropriate amount of drug as much as possible, thereby preventing excessive drug loading from having certain toxic side effects on the human body. That is, the total drug loading is reduced as much as possible while controlling the drug release to ensure that the amount of the drug just meets the demand.

The drug-controlled release layer, drug-loaded layer, and corrosion promoting layer of the present invention each include a polymer, and the thicknesses and molecular weights of the polymers in these three coatings cooperate to control the corrosion of the device matrix, or the device matrix and the metal isolation layer. In some examples provided by the present invention, when the molecular weight of the polymer in the drug-controlled release layer is relatively small, in order to control the overall corrosion rate of the final device matrix, the type and molecular weight of the polymer in the drug-loaded layer and the corrosion control layer may be regulated to meet the requirements, such as increasing the molecular weight of the polymer of the drug-loaded layer and the corrosion control layer, and meanwhile selecting a polymer with a relatively slow degradation rate.

The metal isolation layer introduced in the technical solution of the present invention may be used together with the above-mentioned polymer-containing coatings to control the corrosion rate of the device substrate to ensure that the device is not corroded in the early stage (1-6 months) of implantation, but to allow the device substrate to rapidly degrade after vascular repair is completed. In an in vivo environment, the corrosion control layer and the metal isolation layer are degraded simultaneously, and when the degradable polyester constituting the corrosion control layer is degraded, an acidic substance is generated. When the degradation accumulates to a certain extent, a local acidic environment may be formed, thereby aggravating the corrosion and degradation of the metal isolation layer and the medical device matrix. This process may be controlled through the thickness and molecular weight of the degradable polyester of the corrosion control layer. The lower the molecular weight, the thicker the thickness of the degradable polyester, and the quicker the corrosion and degradation of the metal isolation layer and stent.

In the present invention, the polymers in the corrosion control layer, drug-loaded layer, and drug-controlled release layer cooperate to control the corrosion of the device matrix or the device matrix and the metal isolation layer. Meanwhile, and the corrosion control layer may also serve to isolate the metal ions generated in the corrosion process of the device matrix and the metal isolation layer from the human environment, prevent the unhindered transfer of the metal ions into the vessels of the human body after the metal ions are combined with the active drug, thus causing hemolysis, or prevent excessive metal ions from directly contacting the human body, causing local poisoning or thrombosis.

It should be understood that the terms used herein are for the purpose of describing particular exemplary embodiments only and are not intended to form a limitation. As used herein, the singular forms "a", "an", and "the" are intended to include the plural forms as well, unless the context clearly indicates otherwise. The terms "comprising", "including", "containing", and "having" are inclusive, and therefore specify the presence of stated features, steps, operations, elements, and/or components, but do not preclude the presence or addition of one or more other features, steps, operations, elements, components, and/or combinations thereof. The method steps, processes, and operations described herein are not to be construed as necessarily requiring that they be performed in the particular order described or illustrated, unless the order of performance is explicitly stated. It should also be understood that additional or alternative steps may be used.

**Brief Description of the Drawings**

Various other advantages and benefits will become apparent to a person skilled in the art upon reading the following detailed description of the preferred embodiments. The drawings are only for the purpose of illustrating the preferred embodiments and are not to be construed as limiting the present invention. Moreover, throughout the accompanying drawings, the same components are indicated by the same accompanying symbols.

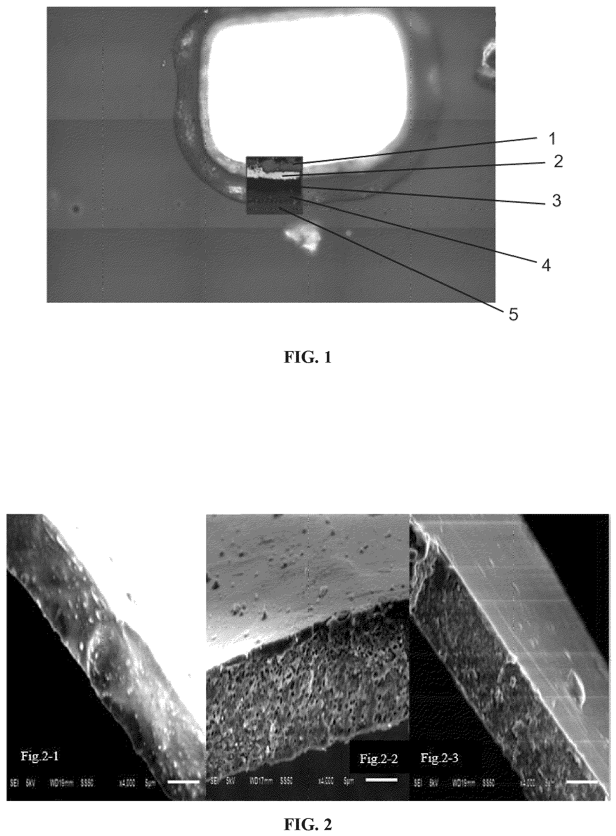

FIG. 1 is a Raman spectrum of tests for the thickness of each layer in a stent coating of example 1, where 1-metal isolation layer, 2-corrosion control layer, 3-drug-loaded layer, 4-drug-controlled release layer, and 5-embedded sample material epoxy polyester;

FIG. 2 is a scanning electron microscope (SEM) graph of testes for particle sizes of drug particles in drug-loaded layers of example 1 and example 6, where FIG. 2-1 shows a cross-section of an ethyl acetate solvent-sprayed coating of example 6 at time zero; FIG. 2-2 shows a cross-section of the ethyl acetate solvent-sprayed coating of example 6 after drug extraction; and FIG. 2-3 shows a cross-section of a chloroform solvent-sprayed coating of example 1 after drug extraction;

FIG. 3 is an XRD graph of tests for the crystallinity of drugs in drug-loaded layers of example 1 and example 6;

FIG. 4 is a graph of drug release rates of coatings of absorbable stents of examples 1-7 and comparative examples 1-5;

FIG. 5 is a graph of mass loss percentages of absorbable stents of examples 1-7 and comparative examples 1-5 in animals; and

FIG. 6 is a graph of tests for the radial support strength of absorbable stents of examples 1-7 and comparative examples 1-5 in animals.

**Detailed Description of the Invention**

The following descriptions are only preferred embodiments of the present invention, and the protection of the present invention is not limited to the following preferred embodiments. For example, in the examples, the description is given by taking a stent as an example, but it does not represent that the technical solution of the present invention is only suitable for a stent. It should be noted that several variations and improvements made by a person skilled in the art based on the present inventive concept are within the scope of the present invention. The reagents or instruments used without indication of the manufacturer are conventional products available commercially.

**Test methods**

1. Determination of weight-average molecular weight of polymer

Detection was performed using a GPC-multi-angle laser light scattering instrument from Wyatt (USA) in combination with molecular weight test systems. The test systems included a liquid-phase pump and a sample injector from Agilent (USA), an Agilent PL MIXED-C GPC column (size: $7.5 \times 300$ mm, 5 $\mu$m) from Agilent (USA), and a multi-angle laser light scattering instrument and a differential detector from Wyatt (USA). The test conditions were as follows: a mobile phase: tetrahydrofuran; a pump flow rate: 1 mL/min; an injection volume: 100 $\mu$L; a laser wavelength: 663.9 nm; and a test temperature: 35°C.

2. Determination of thickness of metal isolation layer

The thickness of the metal isolation layer was determined using an X-ray fluorescence coating thickness gauge method. Firstly, the device was calibrated using a standard block of a corresponding element. After the calibration was completed, a stent sample testing the thickness of the metal isolation layer was fixed on a sample stage and put into the X-ray fluorescence coating thickness gauge. The types of coating and base metal were set, the measurement time was set as 10-15 s, and the thickness was in $\mu$m. OK was clicked to test the thickness of the metal isolation layer.

3. Determination of total thickness of polymer coating

The total thickness was measured using a SEM method. Firstly, the stent sample that was required to test the coating thickness was fixed on a sample stage, and then the sample stage was placed in a JFC-1600 metal spraying device to spray platinum, rotated 180° after spraying once, and then sprayed once again to ensure that all positions were sprayed. The sample sprayed with platinum on the surface was placed into a Buehler room-temperature resin curing agent mixture reagent formulated in the ratio of 5: 1 and removed from a sample sealing shell only after standing still for more than 8 hours. The sealed sample was divided into 3 sections on average and polished using a semi-automatic polishing machine according to a sample polishing procedure. The cross-section of the sample to be measured was polished to a point without grinding marks. The polished sample was fixed on an object stage of the SEM, and the whole object stage was placed in the JFC-1600 metal spraying device for metal spraying for 20 s. The metal-sprayed sample was placed in a JSM-6510 SEM for thickness measurement.

4. Method for testing thickness of each layer of multi-layer drug coating

Firstly, the stent sample that was required to test the characterization of the multi-layer drug coating was fixed on a sample stage, and then the sample stage was placed in a JFC-1600 metal spraying device to spray platinum, rotated 180° after spraying once, and then sprayed once again to ensure that all positions were sprayed. The sample sprayed with platinum on the surface was placed into a Buehler room-temperature resin curing agent mixture reagent formulated in the ratio of 5: 1 and removed from a sample sealing shell only after standing still for more than 8 hours. The sealed sample was divided into 3 sections on average and polished using a semi-automatic polishing machine according to a sample polishing procedure. The cross-section of the sample to be measured was polished to a point without grinding marks. The polished sample was fixed on an object stage of the SEM, and the whole object stage was placed in the JFC-1600 metal spraying device for metal spraying for 20 s.

The cross-section of a coating portion of a multilayer coated medical device was scanned using a micro Raman spectrometer Thermo DXR2 with a laser energy parameter of 6.0 Mw, an exposure time of 20 Hz, exposure times of 50, and a magnification of 500.

Through YZ section scanning, a composition distribution diagram of the cross-section could be obtained. Through characteristic peaks of drug and polylactic acid, the content of drug and polylactic acid in different regions could be qualitatively analyzed, and the thickness of each coating could be tested.

5. Method for testing particle size of drug particles in coating

A coating prepared with different solvents was vacuum-dried at room temperature for 24 hours, put into 5 mL of isopropanol solution, and shaken at a rotation speed of 100 rpm at 37°C for 30 minutes. After the drug was sufficiently extracted by isopropanol, the coating was removed and vacuum-dried for 24 hours, and then put into liquid nitrogen for brittle fracture coating treatment. A sample after the brittle fracture coating treatment was fixed on the object stage of the SEM, and the whole object stage was placed into the JFC-1600 metal spraying device for metal spraying for 20 s. The particle size of the drug particles could be tested by measuring the pore size remaining after drug extraction by placing the metal-sprayed sample in the JSM-6510 SEM.

6. Method for testing crystallinity of drug in coating

Coating solutions prepared with different solvents (ethyl acetate and chloroform) were uniformly sprayed onto stainless steel plates using a spray device and vacuum-dried at room temperature for 24 hours to prepare drug/polymer coating films prepared with different solvents.

An XRD test (Bruker D2 PHASER) was performed on the drug/polymer films prepared with different solvents, and the samples were scanned in an $\theta$-$2\theta$ linkage mode with a scanning angle range of 3-40°, a scanning step of 0.02°, and a scanning speed of 4°/min.

7. Method for testing drug release rate of multilayer coated medical devices in animals

The multilayer coated medical device was implanted into vessels in healthy rabbits, the rabbits were sacrificed at predetermined observation time points, such as 7 hours, 1 day, 3 days, 7 days, 14 days, 28 days, 60 days, 90 days, and 180 days, and the multilayer coated medical device was removed. After the tissue on the medical device was removed as far as possible, the medical device was placed in a brown glass bottle, and an appropriate amount of acetonitrile was added to constant volume. The volume could be determined according to specific specifications to ensure that the stent could be completely immersed in the solution. Ultrasonic treatment was performed for 15 min, and filtering was performed with a 0.22 $\mu$m nylon organic filter membrane. Then, the solution was loaded into a chromatograph injection bottle, and the residual drug amount of the stent was tested using a liquid chromatograph. The drug release amount of the stent was the initial drug amount on the stent minus the residual drug amount (test drug amount) on the stent after immersing, and the drug release percentage was a ratio of the drug release amount to the initial drug amount.

Chromatographic conditions were as follows: acetonitrile: water = 65: 35, the solution being a mobile phase, a flow rate of 1.0 ml/min, a detection wavelength of 278 nm, a column temperature of 50°C, and an injection volume of 10.0 $\mu$l.

8. Test for mass loss of absorbable stent in animals

The multilayer coated medical device was implanted into vessels in healthy rabbits, the rabbits were sacrificed at predetermined observation time points, such as 7 hours, 1 day, 3 days, 7 days, 14 days, 28 days, 60 days, 90 days, and 180 days, and the multilayer coated medical device was removed. After the tissue on the medical device was removed as far as possible, the residual drug amount of the stent was tested using a liquid chromatograph. The stent after the drug amount testing was cleaned using an ethyl acetate solution so that the polymer coating thereon was completely dissolved.

The cleaned stent was examined by micro-CT, and the corrosion of the iron-based matrix was qualitatively analyzed. Later, the stent was placed in a tartaric acid solution for ultrasonic cleaning to remove the surface corrosion product layer, dried, and weighed as $m_{post\text{-}corrosion}$.

The mass loss rate m% of the device was calculated as the difference between the mass $m_{pre\text{-}corrosion}$ of the device before corrosion and the mass $m_{post\text{-}corrosion}$ of the device after corrosion and treatment, divided by the mass $m_{pre\text{-}corrosion}$ of the device before corrosion, and then multiplied by 100%. That is

$$m\% = \frac{m_{pre-corrosion} - m_{post-corrosion}}{m_{pre-corrosion}} \times 100\%.$$

9. Test for radial support strength of stent

The stent was expanded with a suitable balloon (or distal assembly) to a nominal pressure so that an outer diameter of the stent sample reached a given size.

The stent length was measured using a three-dimensional microscope, and then a support force curve test was performed with a compression displacement of 50% as an endpoint. The radial support strength was represented by a numerical value defining a force value per unit length when a nominal diameter of the stent is compressed by 10% as the radial extrusion resistance of the stent (unit: kPa).

**Example 1**

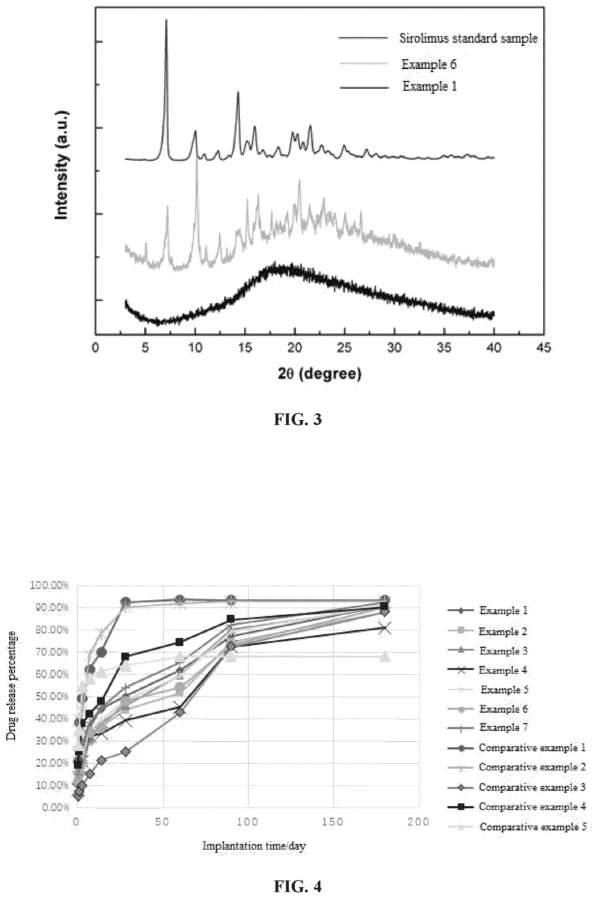

In this example, an iron-based stent with a specification of 3.0 mm × 8 mm was used. A zinc layer with an average thickness of 0.6 $\mu$m was plated on a surface of a substrate by electroplating as a metal isolation layer. A corrosion control layer with an average thickness of 3 $\mu$m was uniformly sprayed on the outside of the metal isolation layer using a stent spraying device. The coating was made of poly(racemic lactic acid) with a molecular weight of 600 kDa, and the solvent was chloroform. After the corrosion control layer was dried, a drug-loaded layer with an average thickness of 5 $\mu$m was uniformly sprayed using the stent spraying device. The coating was made of poly(racemic lactic acid) with a molecular weight of 400 kDa, and the solvent was chloroform. A loaded drug was sirolimus, and a mass ratio of sirolimus to poly(racemic lactic acid) in the drug-loaded layer was 1: 0.5. The drug particles had an average particle size of 0.25 $\mu$m and were amorphous (see FIG. 3). After the drug-loaded layer was dried, a drug-controlled release layer with an average thickness of 3 $\mu$m was uniformly sprayed using the stent spraying device. The coating was made of poly(racemic lactic acid) with a one-month molecular weight retention rate of 74% and a molecular weight of 200 kDa, and the solvent was ethyl acetate.

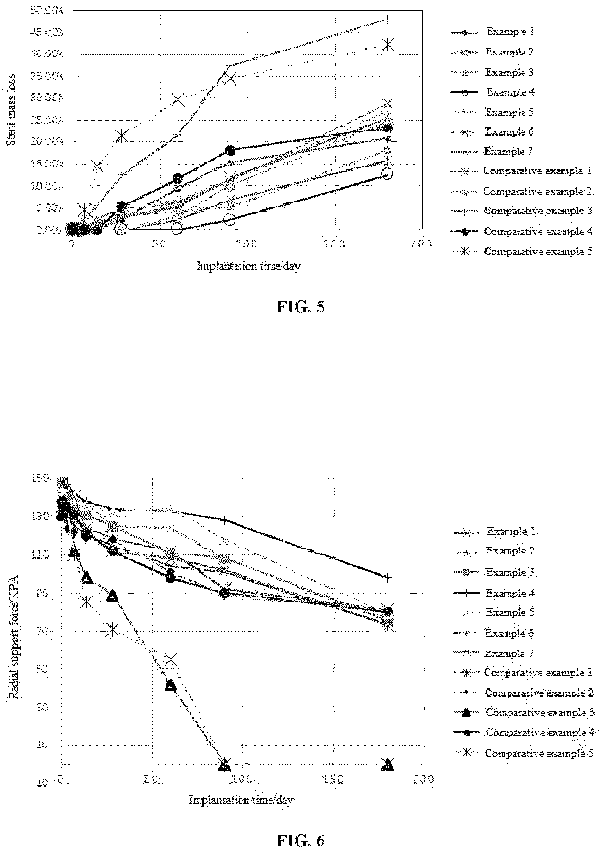

In this example, the in vivo drug release rate of the stent is shown in FIG. 4, the stent mass loss is shown in FIG. 5, and the radial support strength of the stent is shown in FIG. 6. No thrombus was observed in the vessel where the stent was located throughout the implantation period, and the lumen was unblocked without restenosis. The coating ensures that the stent has sufficient support within 6 months and can be completely degraded within 2 years.

**Example 2**

In this example, an iron-based stent with a specification of 3.0 mm × 8 mm was used. A zinc layer with an average thickness of 2 μm was plated on a surface of a substrate by electroplating as a metal isolation layer. A drug-loaded layer with an average thickness of 10 μm was uniformly sprayed on the outside of the metal isolation layer using a stent spraying device. The coating was made of poly(racemic lactic acid) with a molecular weight of 400 kDa, and the solvent was chloroform. A loaded drug was sirolimus, and a mass ratio of sirolimus to poly(racemic lactic acid) in the drug-loaded layer was 1: 8. The drug particles had an average particle size of 0.35 μm and were amorphous. After the drug-loaded layer was dried, a drug-controlled release layer with an average thickness of 1.8 μm was uniformly sprayed using the stent spraying device. The coating was made of poly(racemic lactic acid) with a one-month molecular weight retention rate of 79% and a molecular weight of 400 kDa, and the solvent was chloroform.

In this example, the in vivo drug release rate of the stent is shown in FIG. 4, the stent mass loss is shown in FIG. 5, and the radial support strength of the stent is shown in FIG. 6. No thrombus was observed in the vessel where the stent was located throughout the implantation period, and the lumen was unblocked without restenosis. The coating ensures that the stent has sufficient support within 6 months and can be completely degraded within 2 years.

**Example 3**

In this example, an iron-based stent with a specification of 3.0 mm × 8 mm was used. A zinc layer with an average thickness of 3 μm was plated on a surface of a substrate by electroplating as a metal isolation layer. A corrosion control layer with an average thickness of 6 μm was uniformly sprayed on the outside of the metal isolation layer using a stent spraying device. The coating was made of PLGA (50: 50) with a molecular weight of 50 kDa, and the solvent was chloroform. After the corrosion control layer was dried, a drug-loaded layer with an average thickness of 5 μm was uniformly sprayed using the stent spraying device. The coating was made of PLGA (50: 50) with a molecular weight of 50 kDa, and the solvent was chloroform. A loaded drug was sirolimus, and a mass ratio of sirolimus to PLGA in the drug-loaded layer was 1: 1.25. The drug particles had an average particle size of 0.45 μm and were amorphous. After the drug-loaded layer was dried, a drug-controlled release layer with an average thickness of 6 μm was uniformly sprayed using the stent spraying device. The coating was made of PLGA (50: 50) with a molecular weight of 50 kDa and a one-month molecular weight retention rate of 61%, and the solvent was chloroform.

In this example, the in vivo drug release rate of the stent is shown in FIG. 4, the stent mass loss is shown in FIG. 5, and the radial support strength of the stent is shown in FIG. 6. No thrombus was observed in the vessel where the stent was located throughout the implantation period, and the lumen was unblocked without restenosis. The coating ensures that the stent has sufficient support within 6 months and can be completely degraded within 2 years.

**Example 4**

In this example, an iron-based stent with a specification of 3.0 mm × 8 mm was used. A zinc layer with an average thickness of 3.8 μm was plated on a surface of a substrate by electroplating as a metal isolation layer. A corrosion control layer with an average thickness of 5 μm was uniformly sprayed on the outside of the metal isolation layer using a stent spraying device. The coating was made of poly(racemic lactic acid) with a molecular weight of 400 kDa, and the solvent was ethyl acetate. After the corrosion control layer was dried, a drug-loaded layer with an average thickness of 4 μm was uniformly sprayed using the stent spraying device. The coating was made of poly(racemic lactic acid) with a molecular weight of 200 kDa, and the solvent was ethyl acetate. A loaded drug was sirolimus, and a mass ratio of sirolimus to poly(racemic lactic acid) in the drug-loaded layer was 1: 2. The drug particles had an average particle size of 0.2 μm and were semi-crystalline. After the drug-loaded layer was dried, a drug-controlled release layer with an average thickness of 0.8 μm was uniformly sprayed using the stent spraying device. The coating was made of poly(racemic lactic acid) with a one-month molecular weight retention rate of 81% and a molecular weight of 800 kDa, and the solvent was chloroform.

In this example, the in vivo drug release rate of the stent is shown in FIG. 4, the stent mass loss is shown in FIG. 5, and the radial support strength of the stent is shown in FIG. 6. No thrombus was observed in the vessel where the stent was located throughout the implantation period, and the lumen was unblocked without restenosis. The coating ensures that the stent has sufficient support within 6 months and can be completely degraded within 2 years.

**Example 5**

In this example, an iron-based stent with a specification of 3.0 mm × 8 mm was used. A zinc layer with an average thickness of 1 μm was plated on a surface of a substrate by electroplating as a metal isolation layer. A corrosion control layer with an average thickness of 3.5 μm was uniformly sprayed on the outside of the metal isolation layer using a stent spraying device. The coating was made of poly(racemic lactic acid) with a molecular weight of 200 kDa, and the solvent was ethyl acetate. After the corrosion control layer was dried, a drug-loaded layer with an average thickness of 5 μm was uniformly sprayed using the stent spraying device. The coating was made of poly(racemic lactic acid) with a molecular weight of 100 kDa, and the solvent was ethyl acetate. A loaded drug was sirolimus, and a mass ratio of sirolimus to poly(racemic lactic acid) in the drug-loaded layer was 1: 1.5. The drug particles had an average particle size of 0.7 μm and were semi-crystalline. After the drug-loaded layer was dried, a drug-controlled release layer with an average thickness of 5.8 μm was uniformly sprayed using the stent spraying device. The coating was made of poly(racemic lactic acid) with a molecular weight of 50 kDa and a one-month molecular weight retention rate of 63%, and the solvent was ethyl acetate.

In this example, the in vivo drug release rate of the stent is shown in FIG. 4, the stent mass loss is shown in FIG. 5, and the radial support strength of the stent is shown in FIG. 6. No thrombus was observed in the vessel where the stent was located throughout the implantation period, and the lumen was unblocked without restenosis. The coating ensures that the stent has sufficient support within 6 months and can be completely degraded within 2 years.

**Example 6**

In this example, an iron-based stent with a specification of 3.0 mm × 8 mm was used. A zinc layer with an average thickness of 2.5 μm was plated on a surface of a substrate by electroplating as a metal isolation layer. A corrosion control layer with an average thickness of 2.5 μm was uniformly sprayed on the outside of the metal isolation layer using a stent spraying device. The coating was made of poly(racemic lactic acid) with a molecular weight of 300 kDa, and the solvent was ethyl acetate. After the corrosion control layer was dried, a drug-loaded layer with an average thickness of 8 μm was uniformly sprayed using the stent spraying device. The coating was made of poly(racemic lactic acid) with a molecular weight of 300 kDa, and the solvent was ethyl acetate. A loaded drug was sirolimus, and a mass ratio of sirolimus to poly(racemic lactic acid) in the drug-loaded layer was 1: 6. The drug particles had an average particle size of 0.35 μm and were semi-crystalline (see FIG. 3). After the drug-loaded layer was dried, a drug-controlled release layer with an average thickness of 2.6 μm was uniformly sprayed using the stent spraying device, where the molecular weight was 600 kDa, the one-month molecular weight retention rate was 80%, and the solvent was chloroform.

In this example, the in vivo drug release rate of the stent is shown in FIG. 4, the stent mass loss is shown in FIG. 5, and the radial support strength of the stent is shown in FIG. 6. No thrombus was observed in the vessel where the stent was located throughout the implantation period, and the lumen was unblocked without restenosis.

**Example 7**

In this example, an iron-based stent with a specification of 3.0 mm × 8 mm was used. A zinc layer with an average thickness of 1.5 μm was plated on a surface of a substrate by electroplating as a metal isolation layer. A corrosion control layer with an average thickness of 2 μm was uniformly sprayed on the outside of the metal isolation layer using a stent spraying device. The coating was made of poly(racemic lactic acid) with a molecular weight of 200 kDa, and the solvent was acetone. After the corrosion control layer was dried, a drug-loaded layer with an average thickness of 3 μm was uniformly sprayed using the stent spraying device. The coating was made of poly(racemic lactic acid) with a molecular weight of 600 kDa, and the solvent was acetone. A loaded drug was sirolimus, and a mass ratio of sirolimus to poly(racemic lactic acid) in the drug-loaded layer was 1: 1.5. The drug particles had an average particle size of 0.6 μm. After the drug-loaded layer was dried, a drug-controlled release layer with an average thickness of 6.6 μm was uniformly sprayed using the stent spraying device, where the molecular weight was 100 kDa, the one-month molecular weight retention rate was 65%, and the solvent was acetone.

In this example, the in vivo drug release rate of the stent is shown in FIG. 4, the stent mass loss is shown in FIG. 5, and the radial support strength of the stent is shown in FIG. 6. No thrombus was observed in the vessel where the stent was located throughout the implantation period, and the lumen was unblocked without restenosis. The coating ensures that the stent has sufficient support within 6 months and can be completely degraded within 2 years.

**Comparative example 1**

In this example, an iron-based stent with a specification of 3.0 mm × 8 mm was used. A zinc layer with an average thickness of 1 μm was plated on a surface of a substrate by electroplating as a metal isolation layer. A corrosion control layer with an average thickness of 3 μm was uniformly sprayed on the outside of the metal isolation layer using a stent spraying device. The coating was made of poly(racemic lactic acid) with a molecular weight of 400 kDa, and the solvent was ethyl acetate. After the corrosion control layer was dried, a drug-loaded layer with an average thickness of 5 μm was uniformly sprayed using the stent spraying device. The coating was made of poly(racemic lactic acid) with a molecular weight of 200 kDa, and the solvent was ethyl acetate. A loaded drug was sirolimus, and a mass ratio of sirolimus to poly(racemic lactic acid) in the drug-loaded layer was 1: 2. The drug particles had an average particle size of 0.3 μm and were semi-crystalline.

In this comparative example, the in vivo drug release rate of the stent is shown in FIG. 4. It can be seen from FIG. 4 that in this comparative example, the drug in the stent experiences a burst release in the early stage, with most of the drug released in the early stage of stent implantation, resulting in insufficient drug release in the middle and late stages. On the 90th day, there is a relatively severe sign of proliferation in the vessel where the stent is located. The stent mass loss results are shown in FIG. 5, and the radial support strength of the stent is shown in FIG. 6. The stent has a normal corrosion rate in vivo and can still provide effective support in the 6th month.

## Comparative example 2

In this example, an iron-based stent with a specification of 3.0 mm × 8 mm was used. A zinc layer with an average thickness of 1.5 $\mu$m was plated on a surface of a substrate by electroplating as a metal isolation layer. A corrosion control layer with an average thickness of 5 $\mu$m was uniformly sprayed on the outside of the metal isolation layer using a stent spraying device. The coating was made of poly(racemic lactic acid) with a molecular weight of 200 kDa, and the solvent was chloroform. After the corrosion control layer was dried, a drug-loaded layer with an average thickness of 5 $\mu$m was uniformly sprayed using the stent spraying device. The coating was made of poly(racemic lactic acid) with a molecular weight of 200 kDa, and the solvent was chloroform. A loaded drug was sirolimus, and a mass ratio of sirolimus to poly(racemic lactic acid) in the drug-loaded layer was 1: 1.25. The coating was made of poly(racemic lactic acid) with a molecular weight of 400 kDa, and the solvent was chloroform. The loaded drug was sirolimus. The drug particles had an average particle size of 0.35 $\mu$m and were amorphous.

In this comparative example, the in vivo drug release rate of the stent is shown in FIG. 4. It can be seen from FIG. 4 that in this comparative example, the drug in the stent experiences a burst release in the early stage, with most of the drug released in the early stage of stent implantation, resulting in insufficient drug release in the middle and late stages. On the 90th day, relatively severe signs of proliferation and thrombosis are observed in the vessel where the stent is located.

The stent mass loss rates are shown in FIG. 5, and the radial support strength of the stent is shown in FIG. 6. The stent has a normal corrosion rate in vivo and can still provide effective support in the 6th month.

## Comparative example 3

In this example, an iron-based stent with a specification of 3.0 mm × 8 mm was used. A zinc layer with an average thickness of 1 $\mu$m was plated on a surface of a substrate by electroplating as a metal isolation layer. A corrosion control layer with an average thickness of 4 $\mu$m was uniformly sprayed on the outside of the metal isolation layer using a stent spraying device. The coating was made of poly(racemic lactic acid) with a molecular weight of 800 kDa, and the solvent was chloroform. After the corrosion control layer was dried, a drug-loaded layer with an average thickness of 4 $\mu$m was uniformly sprayed using the stent spraying device. The coating was made of poly(racemic lactic acid) with a molecular weight of 400 kDa. A loaded drug was sirolimus, and a mass ratio of sirolimus to poly(racemic lactic acid) in the drug-loaded layer was 1: 2. The drug particles had an average particle size of 0.35 $\mu$m and were semi-crystalline. The solvent was ethyl acetate. After the drug-loaded layer was dried, a drug-controlled release layer with an average thickness of 15 $\mu$m was uniformly sprayed using the stent spraying device. The coating was made of poly(racemic lactic acid) with a one-month molecular weight retention rate of 74% and a molecular weight of 200 kDa, and the solvent was ethyl acetate.

In this comparative example, the in vivo drug release rate of the stent is shown in FIG. 4. It can be seen from FIG. 4 that in this comparative example, the stent has insufficient drug release in the early stage and cannot play a therapeutic role in the early stage of implantation. On the 14th day, there is a sign of thrombosis in the vessel where the stent is located.

The stent mass loss is shown in FIG. 5, and the radial support strength of the stent is shown in FIG. 6. As the coating thickness increases, the amount of polylactic acid also correspondingly increases, resulting in an accelerated corrosion rate of the stent in vivo. The drug release rate is slow in the early stage, the normal form of the stent cannot be maintained after implantation for 60 days, and effective support cannot be provided. There is an obvious thrombosis on the stent rod at day 30, and after 90 days of stent implantation, the vascular restenosis is severe, with a loss of lumen area exceeding 60%.

## Comparative example 4

In this example, an iron-based stent with a specification of 3.0 mm × 8 mm was used. A zinc layer with an average thickness of 0.6 $\mu$m was plated on a surface of a substrate by electroplating as a metal isolation layer. A corrosion control layer with an average thickness of 2.5 $\mu$m was uniformly sprayed on the outside of the metal isolation layer using a stent spraying device. The coating was made of poly(racemic lactic acid) with a molecular weight of 200 kDa, and the solvent was chloroform. After the corrosion control layer was dried, a drug-loaded layer with an average thickness of 8 $\mu$m was uniformly sprayed using the stent spraying device. The coating was made of poly(racemic lactic acid) with a molecular weight of 200 kDa, and the solvent was chloroform. A loaded drug was sirolimus, and a mass ratio of sirolimus to poly(racemic lactic acid) in the drug-loaded layer was 1: 8. The drug particles had an average particle size of 0.35 μm and were amorphous. After the drug-loaded layer was dried, a drug-controlled release layer with an average thickness of 0.2 μm was uniformly sprayed using the stent spraying device. The coating was made of poly(racemic lactic acid) with a molecular weight of 100 kDa and a one-month molecular weight retention rate of 68%, and the solvent was ethyl acetate.

In this comparative example, the in vivo drug release rate of the stent is shown in FIG. 4, the stent mass loss is shown in FIG. 5, and the radial support strength of the stent is shown in FIG. 6. The corrosion rate of the stent in vivo is normal, and the stent can still provide effective support in the 6th month. However, since the drug-controlled release layer of the stent in this comparative example is too thin, the drug experiences a burst release in the early stage, with most of the drug released in the early stage of stent implantation, resulting in insufficient drug release in the middle and late stages. On the 90th day, there is a relatively severe sign of proliferation in the vessel where the stent is located.

**Comparative example 5**

In this example, an iron-based stent with a specification of 3.0 mm × 8 mm was used. A corrosion control layer with an average thickness of 2.5 μm was uniformly sprayed on the surface of a metal substrate using a stent spraying device. The coating was made of poly(racemic lactic acid) with a molecular weight of 100 kDa, and the solvent was chloroform. After the corrosion control layer was dried, a drug-loaded layer with an average thickness of 5 μm was uniformly sprayed using the stent spraying device. The coating was made of poly(racemic lactic acid) with a molecular weight of 100 kDa, and the solvent was chloroform. A loaded drug was sirolimus, and a mass ratio of sirolimus to poly(racemic lactic acid) in the drug-loaded layer was 1: 0.5. The drug particles had an average particle size of 0.25 μm and were amorphous. After the drug-loaded layer was dried, a drug-controlled release layer with an average thickness of 0.2 μm was uniformly sprayed using the stent spraying device. The coating was made of poly(racemic lactic acid) with a molecular weight of 100 kDa and a one-month molecular weight retention rate of 68%, and the solvent was ethyl acetate.

In this comparative example, the in vivo drug release rate of the stent is shown in FIG. 4, the stent mass loss is shown in FIG. 5, and the radial support strength of the stent is shown in FIG. 6. Due to the lack of a metal isolation layer, the stent matrix is corroded too quickly, and the stent rod is fractured at the early stage of implantation, resulting in accelerated drug release. Most of the drug is released in the early stage of stent implantation. The normal form of the stent cannot be maintained after implantation for 60 days, and effective support cannot be provided. There is an obvious thrombosis on the stent rod at day 30, the color of the tissue around the stent rod is abnormal, and after 90 days of stent implantation, the vascular restenosis is severe, with a loss of lumen area exceeding 60%.

As can be seen from the above, in comparison with example 4 and comparative example 1, the burst release of the drug occurs in comparative example 1 due to the lack of a drug-controlled release layer, and a large amount of drug is released in a short period of time, resulting in very little drug release and low drug utilization over a relatively long period in the middle and late stages.

Although comparative example 1 has the same drug-polyester mass ratio as in example 4, due to the lack of a drug-controlled release layer in comparative example 1, a large amount of drug is released in a short period of time, resulting in very little drug release and low drug utilization over a relatively long period in the middle and late stages. In comparison with comparative example 2, the drug particle morphology and solvent type in comparative example 2 are different. However, due to the lack of a drug-controlled release layer, the burst release of the drug in vivo occurs.

In comparison with example 4 and comparative example 3, although they have the same solvent and drug particle morphology, the drug-controlled release layer in comparative example 3 is too thick so that the drug release in the early and middle period is insufficient, resulting in vascular proliferation and restenosis, which also does not play a role in drug treatment. In comparative example 3, the polylactic acid coating is too thick, and the content of polylactic acid is increased, resulting in an accelerated corrosion rate of the stent in vivo. The normal form of the stent cannot be maintained after implantation for 60 days, and effective support cannot be provided, which aggravates the symptoms of vascular thrombosis and restenosis.

The drug-controlled release layer is provided in both example 4 and comparative example 5. However, since the thickness of the drug-controlled release layer is insufficient, a large amount of drug is released in a short time as well after degradation of surface polylactic acid, resulting in very little drug release and low drug utilization over a relatively long period in the middle and late stages.

However, in comparative example 5, due to the lack of a metal isolation layer and the lack of corresponding polymer type and polymer molecular weight, the polymer is directly in contact with the stent substrate, and the stent matrix corrodes too quickly. The stent rod is fractured at the early stage of implantation, resulting in accelerated drug release. The normal form of the stent cannot be maintained after implantation for 60 days, and effective support cannot be provided.

However, in examples 1-7, although the molecular weights, polymer materials, reagents, crystallinity, and particle sizes of drug particles are different, after formulating a drug-controlled release layer with a suitable thickness, burst release of the drug does not occur in the early stage, and the drug is suitably released during the release period, which is suitably matched with the degradation period of the stent substrate.

Numerous other embodiments of the present invention are possible. Without departing from the spirit of the present invention and the essence thereof, a person skilled in the art may make various corresponding changes and deformations in accordance with the present invention, and these corresponding changes and deformations shall fall within the scope of the claims appended to the present invention.

**Claims**

1. A coating for a medical device, wherein the coating comprises a drug-loaded layer and a drug-controlled release layer covering the drug-loaded layer, and each of the drug-loaded layer and the drug-controlled release layer comprises a polymer, wherein a molecular weight retention rate m of the polymer in the drug-controlled release layer, a weight-average molecular weight $Mw_{controlled\ release}$ of the polymer, and a thickness $X_{controlled\ release}$ of the polymer meet the following relational expression:

$$\mathrm{k} \times \frac{\sqrt{\left|\frac{A_1+A_2}{Mw_{controlled\ release}+A_2}-1\right|}}{m} - b \leq X_{controlled\ release} \leq \mathrm{k} \times$$

$$\frac{\sqrt{\left|\frac{A_1+A_2}{Mw_{controlled\ release}+A_2}-1\right|}}{m} + b;$$

wherein $A_1$ is a fitting constant of 823;
$A_2$ is a fitting constant of 79.9;
k is a fitting constant of 1.5;
b is a fitting constant with a range of $0 \leq b \leq 2$;
$Mw_{controlled\ release}$ is the weight-average molecular weight of the polymer in the drug-controlled release layer in kDa, and $X_{controlled\ release}$ is in $\mu$m.

2. The coating for a medical device according to claim 1, wherein the weight-average molecular weight $Mw_{controlled\ release}$ of the polymer in the drug-controlled release layer is 20-900 kDa; the molecular weight retention rate m of the polymer is 50%-80%.

3. The coating for a medical device according to claim 1, wherein a drug-to-polymer mass ratio in the drug-loaded layer is 1: (0.1-50).

4. The coating for a medical device according to claim 1, wherein drug particles in the drug-loaded layer have a particle size $r \leq 0.8\ \mu$m.

5. The coating for a medical device according to claim 1, wherein a sum of thicknesses of the drug-loaded layer and the drug-controlled release layer is not less than 2.5 $\mu$m.

6. The coating for a medical device according to claim 1, wherein the polymer in the drug-loaded layer has a molecular weight of 30-1,000 kDa.

7. The coating for a medical device according to claim 1, wherein an inner side of the drug-loaded layer further comprises a corrosion control layer; a polymer in the corrosion control layer has a molecular weight of 50-1,000 kDa.

8. The coating for a medical device according to claim 7, wherein a total thickness of the corrosion control layer, the drug-loaded layer, and the drug-controlled release layer is 3.5-45 $\mu$m.

9. The coating for a medical device according to claim 7, wherein a thickness ratio of the corrosion control layer, the drug-loaded layer, and the drug-controlled release layer is 1: (0.5-15): (0.2-13).

10. The coating for a medical device according to claim 7, wherein the coating further comprises a metal isolation layer located on an inner side the corrosion control layer.

11. The coating for a medical device according to claim 10, wherein a thickness of the metal isolation layer is $\geq 0.6\ \mu$m.

**12.** The coating for a medical device according to claim 1, wherein the polymer is at least one of a degradable polyester or a degradable polyanhydride; the polymer is selected from at least one of poly(racemic lactic acid), poly(L-lactic acid), poly(D-lactic acid), poly(hydroxyethyl ester), polyurethane, polyamino acid, poly(lactic-co-diglycolic acid), poly-D,L-lactide, polypropylene glycol, polyglycolic acid, poly(lactic-co-glycolic acid) (PLGA), polysalicylic anhydride ester, polytrimethylene carbonate, polycaprolactone, poly($\varepsilon$-caprolactone), polyhydroxyalkanoate, polyacrylate, polysuccinate, poly($\beta$-hydroxybutyrate), polyethylene glycol adipate, poly(1,3-bis(p-carboxyphenoxy)propane-sebacic acid), polyerucic acid dimer-sebacic acid, and polyfumaric acid-sebacic acid.

**13.** The coating for a medical device according to claim 1, wherein a drug loaded in the drug-loaded layer is at least one of a drug inhibiting vascular proliferation, an anti-inflammatory drug, an antithrombotic drug, and a sensitizing drug.

**14.** The coating for a medical device according to claim 1, wherein a drug loaded in the drug-loaded layer is at least one of paclitaxel, rapamycin, cilostazol, heparin, dexamethasone, tacrolimus, everolimus, calcium gluconate, chlorpheniramine, or cortisone.

**15.** The coating for a medical device according to claim 1, wherein the medical device is a degradable medical device; the medical device is a degradable metal medical device or a degradable non-metal medical device.

**16.** The coating for a medical device according to claim 1, wherein a degradable medical device comprises any one of a vascular stent, a heart valve, a non-vascular endoluminal stent, an occluder, an orthopedic implant, a dental implant, a respiratory implant, a gynecological implant, an andrological implant, a suture, or a bolt.

**FIG. 1**

**FIG. 2**

FIG. 3

FIG. 4

**FIG. 5**

**FIG. 6**

## INTERNATIONAL SEARCH REPORT

| International application No. |
|---|
| **PCT/CN2023/110241** |

### A. CLASSIFICATION OF SUBJECT MATTER

A61L31/10(2006.01)i

According to International Patent Classification (IPC) or to both national classification and IPC

### B. FIELDS SEARCHED

Minimum documentation searched (classification system followed by classification symbols)

IPC: A61L

Documentation searched other than minimum documentation to the extent that such documents are included in the fields searched

Electronic data base consulted during the international search (name of data base and, where practicable, search terms used)

CNABS; CNTXT; CNKI; WPABSC; ENTXTC; WPABS; DWPI; ENTXT; ISI Web of Science: 元心科技, 李云飞, 张德元, 齐海萍, 张万谦, 李海锋, 重均分子量, 分子量保留率, 分子量保存率, 分子量保持率, 分子量减失率, 分子量损失率, 分子量减少率, 分子量减小率, 分子量降低率, 控释层, 控制释放层, 控制层, 控释涂层, 控制释放涂层, 控制涂层, 厚度, 控释, 控制释放, 控制, 药物, 医疗器械, 医用器械, 植入物, weight-average molecular weight, weight average molecular weight, molecular weight, retention, reservation, conservation, preserving, lost, reduction, reduce?, decrease, releas???, control???, coating, layer, thickness, medical, apparatus, instruments, device, implant, implantion, medicine, therapy, drug, active agent

### C. DOCUMENTS CONSIDERED TO BE RELEVANT

| Category* | Citation of document, with indication, where appropriate, of the relevant passages | Relevant to claim No. |
|---|---|---|
| Y | CN 105944155 A (LEPU MEDICAL TECHNOLOGY (BEIJING) CO., LTD.) 21 September 2016 (2016-09-21) description, paragraphs [0012]-[0044] | 1-16 |
| Y | CN 113116595 A (YUANXIN TECHNOLOGY (SHENZHEN) CO., LTD.) 16 July 2021 (2021-07-16) description, paragraphs [0030]-[0056] | 1-16 |
| Y | CN 205698633 U (LEPU MEDICAL TECHNOLOGY (BEIJING) CO., LTD.) 23 November 2016 (2016-11-23) description, paragraphs [0012]-[0045] | 1-16 |
| A | CN 101181650 A (SHANGHAI PUTUO DISTRICT CENTER HOSPITAL) 21 May 2008 (2008-05-21) entire document | 1-16 |

☑ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 October 2023** | **26 October 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/110241** |

**C. DOCUMENTS CONSIDERED TO BE RELEVANT**

| Category* | Citation of document, with indication, where appropriate, of the relevant passages | Relevant to claim No. |
| --- | --- | --- |
| A | CN 1678359 A (CORDIS CORP.) 05 October 2005 (2005-10-05)<br>entire document | 1-16 |
| A | US 2011276148 A1 (DEPUY INTERNATIONAL LTD.) 10 November 2011 (2011-11-10)<br>entire document | 1-16 |

Form PCT/ISA/210 (second sheet) (July 2022)

## INTERNATIONAL SEARCH REPORT
### Information on patent family members

International application No.

**PCT/CN2023/110241**

| Patent document cited in search report | | | Publication date (day/month/year) | Patent family member(s) | | | Publication date (day/month/year) |
|---|---|---|---|---|---|---|---|
| CN | 105944155 | A | 21 September 2016 | None | | | |
| CN | 113116595 | A | 16 July 2021 | CN | 113116595 | B | 21 June 2022 |
| CN | 205698633 | U | 23 November 2016 | None | | | |
| CN | 101181650 | A | 21 May 2008 | CN | 101181650 | B | 16 June 2010 |
| CN | 1678359 | A | 05 October 2005 | EP | 1534357 | A2 | 01 June 2005 |
| | | | | WO | 2004002547 | A2 | 08 January 2004 |
| | | | | US | 2005113907 | A1 | 26 May 2005 |
| | | | | MXPA | 05000203 | A | 30 September 2005 |
| | | | | JP | 2005537044 | A | 08 December 2005 |
| | | | | CA | 2490898 | A1 | 08 January 2004 |
| | | | | US | 2004002755 | A1 | 01 January 2004 |
| | | | | AU | 2003253696 | A1 | 19 January 2004 |
| US | 2011276148 | A1 | 10 November 2011 | US | 9849220 | B2 | 26 December 2017 |
| | | | | CA | 2740854 | A1 | 22 April 2010 |
| | | | | CA | 2740854 | C | 03 July 2018 |
| | | | | GB | 0818933 | D0 | 19 November 2008 |
| | | | | AU | 2009305353 | A1 | 22 April 2010 |
| | | | | AU | 2009305353 | B2 | 20 November 2014 |
| | | | | JP | 2012505678 | A | 08 March 2012 |
| | | | | JP | 5507570 | B2 | 28 May 2014 |
| | | | | WO | 2010043710 | A2 | 22 April 2010 |
| | | | | KR | 20110074771 | A | 01 July 2011 |
| | | | | KR | 101643543 | B1 | 29 July 2016 |
| | | | | EP | 2349373 | A2 | 03 August 2011 |
| | | | | ZA | 201103535 | B | 26 March 2014 |
| | | | | US | 2018140751 | A1 | 24 May 2018 |
| | | | | US | 10646623 | B2 | 12 May 2020 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107496996 B